(19) Europäisches Patentamt
European Patent Office
Office européen des brevets

(11) Publication number: 0 086 647 B1

(12) EUROPEAN PATENT SPECIFICATION

(45) Date of publication of patent specification: 17.09.86

(21) Application number: 83300710.7

(22) Date of filing: 11.02.83

(51) Int. Cl.[4]: C 07 D 239/36, C 07 D 405/12, C 07 D 417/12, A 61 K 31/505, C 07 D 417/14, C 07 C 123/00, C 07 C 69/716, C 07 C 69/738

(54) **Pyrimidone compounds, their preparation, and pharmaceutical compositions containing them.**

(30) Priority: 15.02.82 JP 22280/82
26.08.82 JP 148123/82
27.08.82 JP 148967/82
01.09.82 JP 151997/82
13.12.82 JP 218209/82

(43) Date of publication of application:
24.08.83 Bulletin 83/34

(45) Publication of the grant of the patent:
17.09.86 Bulletin 86/38

(84) Designated Contracting States:
AT BE CH DE FR GB IT LI LU NL SE

(56) References cited:
EP-A-0 003 677
EP-A-0 015 138
EP-A-0 060 697

MEDICINAL CHEMISTRY BY BURGER, 3RD EDITION, P. 75

**The file contains technical information submitted after the application was filed and not included in this specification**

(73) Proprietor: YAMANOUCHI PHARMACEUTICAL CO. LTD.
No. 5-1 Nihonbashi-Honcho, 2-chome Chuo-ku
Tokyo (JP)

(72) Inventor: Yanagisawa, Isao
No. 2-22-8, Shakujiidai
Nerima-ku Tokyo (JP)
Inventor: Ohta, Mitsuaki
No. 3-16-1, Hasune
Itabashi-ku Tokyo (JP)
Inventor: Takagi, Tokuichi
No. 8-27-13, Takasago
Katsushika-ku Tokyo (JP)

(74) Representative: Geering, Keith Edwin et al
REDDIE & GROSE 16 Theobalds Road
London WC1X 8PL (GB)

EP 0 086 647 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to pyrimidone compounds useful in general as gastric acid secretion inhibitors, their preparation and intermediates therefor, and medical compositions containing them. Some compounds of this invention are also useful as anti-inflammatory agents or medicaments for treating disease of the cardiovascular system or disease of the stomach.

Known pyrimidone compounds include those of the formula:

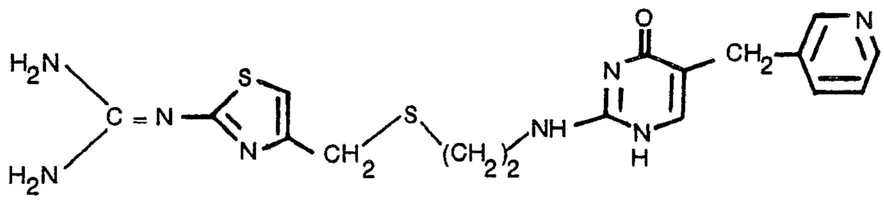

which exhibit prolonged gastric acid secretion inhibiting activity (published Japanese Patent Application No. 55—115883 (EP—A—15138)); and those of the formula:

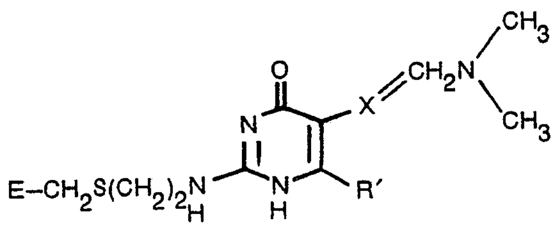

where

E is

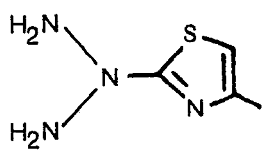

or

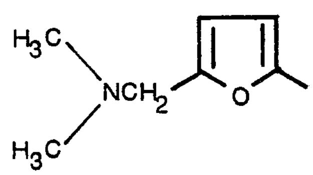

and X is —$CH_2$-heterocyclic, which have the characteristic of a dimethylaminomethyl group at the 5-position. Other pyrimidone derivatives are disclosed in published Japanese patent applications Nos. 54—115385 (EP—A—3677) and 55—500898.

According to this invention there are provided pyrimidone compounds of general formula I:

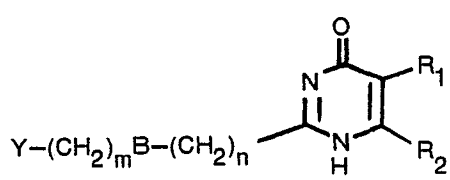

I

wherein $R_1$ represents the group

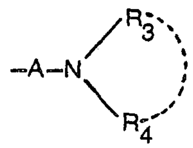

wherein A represents a lower alkylene group and $R_3$ and $R_4$ are the same or different lower alkyl groups or combine to form with the adjacent nitrogen atom a piperidine or pyrrolidine ring; $R_2$ represents a hydrogen atom, a lower alkyl group or a trifluoromethyl group; Y represents a heterocyclic group of the formula:

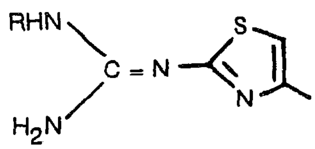

,

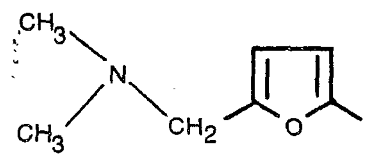

or

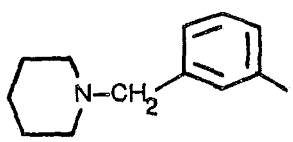

wherein R represents a hydrogen atom or a lower alkyl group; B represents an oxygen atom or a sulfur atom; m represents zero or an integer of 1 to 3; and n represents an integer of 1 to 3; and the pharmacologically acceptable salts thereof. Symbols herein have the same significance throughout unless otherwise indicated.

The invention also provides processes for preparing compounds of general formula I, and pharmaceutical compositions containing effective amounts of formula I compounds and pharmaceutically acceptable carrier(s) and/or diluent(s).

The term "lower" in the above definition means a straight or branched carbon chain having 1 to 5 carbon atoms. For example, as lower alkyl groups, there are methyl ethyl, propyl, isopropyl, butyl, isobutyl, sec-bytyl, tert-butyl, and pentyl groups as lower alkenyl groups there are vinyl and allyl groups; as lower alkynyl groups there are ethynyl, 2-propynyl, 3-butynyl, 2-butynyl, 1-butynyl, 1-methyl-2-propynyl, and pentynyl groups; and as lower alkylene groups there are methylene, ethylene, methmethylene, trimethylene, propylene, tetramethylene, methyltrimethylene, and pentamethylene groups.

Preferred combinations of the meanings of the above symbols are:— B is oxygen and m is zero; B is sulfur and m and n are the same or different integers of 1 to 3; Y is

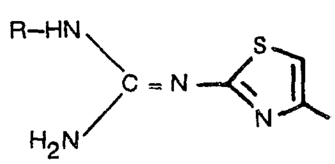

,

B is sulfur and m and n are the same or different integers of 1 to 3; R is a $C_1$ to $C_3$ alkyl group; Y is

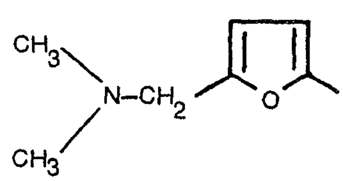

and m and n are the same or different integers of 1 to 3; Y is

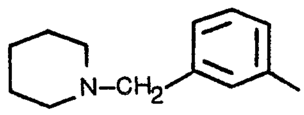

and B is oxygen and m is zero.

Typical compounds of this invention are as follows:

2-[2-[[[2-[(diaminomethylene)amino]-4-thiazolyl]methyl]thio]ethyl]-6-methyl-5-(2-methyl-3-dimethylaminopropyl)-4(1H)-pyrimidone;

5-(3-diethylaminopropyl)-6-methyl-2-[2-[[[2-[[-n-propyldiaminomethylene]amino]-4-thiazolyl]methyl]thio]ethyl]-4(1H)-pyrimidone;

2-[2-[[[2-[(diaminomethylene)amino]-4-thiazolyl]methyl]thio]ethyl]-5-(3-dimethylaminopropyl)-6-methyl-4(1H)-pyrimidone;

2-[2-[[[2-[(diaminomethylene)amino]-4-thiazolyl]methyl]thio]ethyl]-5-(3-diethylaminopropyl)-6-methyl-4(1H)-pyrimidone.

The compounds of formula I can form acid additions salts, with mineral acids such as hydrochloric acid, hydrobromic acid, hydroiodic acid and sulfuric acid, and with organic acids such as maleic acid, fumaric acid, and picric acid. Furthermore, there also exist tautomers of the compounds of formula I at the 1H- and 3H-positions, and there exist occasionally hydroxy-tautomers in a small proportion. The invention includes such salts and tautomers.

The compounds of formula I and their acid addition salts have histamine $H_2$-receptor blocking activity, and are useful as gastric acid secretion inhibitors; some additionally have histamine $H_1$-receptor blocking activity and are useful also be anti-inflammatory agents and as medicaments acting on the cardio-vascular system.

For many of the compounds of this invention, their activities, such as histamine $H_2$-receptor blocking activity and/or histamine $H_1$-receptor blocking activity, are prolonged, lasting for a long period of time.

The compounds of this invention can be administered orally or parenterally, as the free bases or pharmacologically acceptable salts thereof. They are generally used as medical or pharmaceutical compositions with suitable carriers or diluent. The rate of administration is usually 100 to 800 mg per day for adults in 1 to 4 doses.

It has been shown by Shay rat 4 hr method (Shay et al., Gastroenterology, *5,* pages 43—61, 1945) and other animal experiment methods that compounds of this invention inhibit gastric acid secretion at administered doses below 10—50 mg/kg and have histamine $H_2$-receptor blocking activity; and that some of the compounds also have histamine $H_1$-receptor blocking activity.

Some relevant data is shown in the following Table:

TABLE

| Compound | $H_2$-blocking activity $ED_{50}$ | Acid Inhibition — Shay (4hr.) (mg./kg.) (Dog) |
|---|---|---|
| 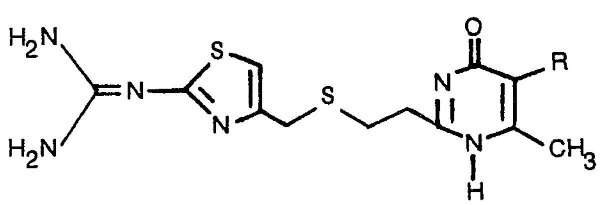 | | |
| R=dimethylaminopropyl | $3.4 \times 10^{-7}$ | |
| R=diethylaminopropyl | $5 \times 10^{-7}$ | 83% (3 mg) |

Compounds of this invention can be produced by various processes, some of which are shown below:

Process I.

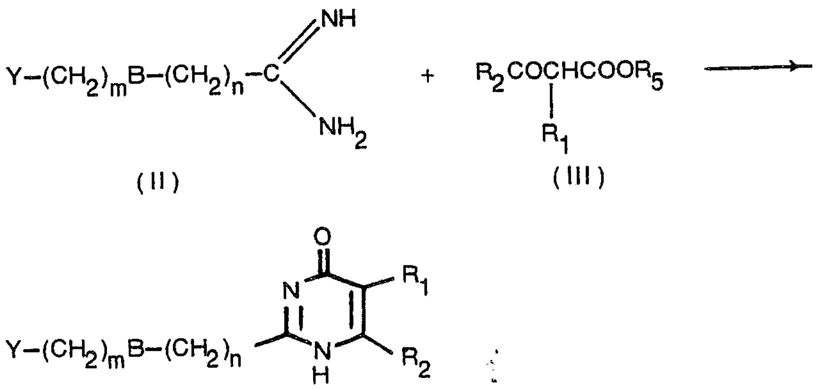

(II) (III)

(I)

(wherein $R_5$ represents a lower alkyl group)

This process is a condensation cyclization reaction between the amidine compound of formula (II) and the acylacetic acid derivative of formula (III).

The reaction is usually performed in a solvent at room temperature or under heating. As the solvent, there is preferably used alcohol (e.g. methanol, ethanol, isopropanol, dimethylformamide, dimethylsulfoxide, methylcellosolve, ethylcellosolve or diglyme. If necessary, a basic material may be present, suitable base materials including alkali metal (e.g. sodium or potassium) alcoholate, sodium hydroxide.

The starting materials (II) and (III) may be used in equal molar amounts, or some excess of either may be used.

Process II

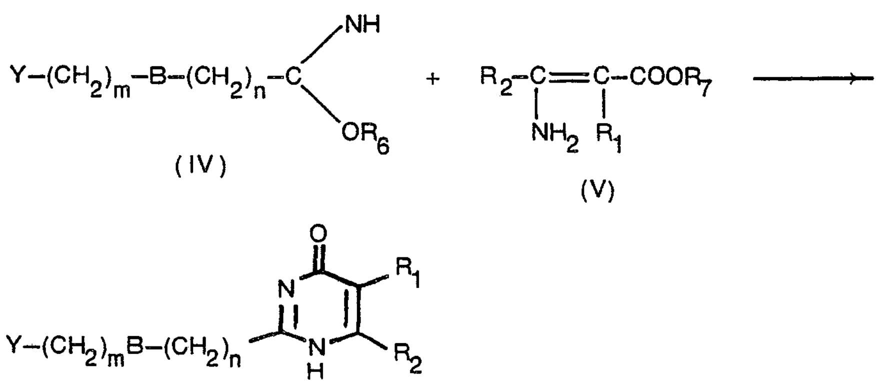

(IV) (V)

(I)

(wherein $R_6$ and $R_7$ are the same or different alkyl groups).

This process is a condensation cyclization reaction between the imidate compound of formula IV and the 3-aminoacrylic acid ester derivative of formula V. The reaction can be performed substantially as for Process I — i.e. using the same organic solvents and if necessary the same basic materials and reacting with heating or under reflux.

The compounds of this invention thus prepared can be separated as the free base or an acid addition salt thereof, and further can be purified by standard procedures such as column chromatography or recrystallization.

The starting materials of formula II and IV can be obtained as disclosed in published Japanese patent applications Nos. 55—115860, 55—115877 and 55—118476. Some of the starting materials of formula II and III are novel, and can be produced as described in the Reference Examples 1 to 8 below.

Compounds of formula I and salts thereof, and their preparation, are illustrated by Examples 1 to 7.

Herein mp., Anal., NMR and Mass. are abbreviations for melting point, elementary analysis values, nuclear magnetic resonance spectrum and mass spectrum, respectively.

Reference Example 1

Ethyl α-(3-pyridylmethyl)acetoacetate

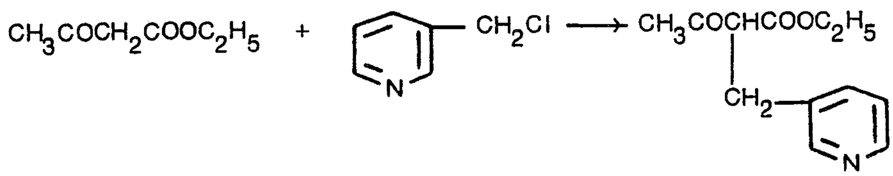

Under nitrogen, 8.4 g of metallic sodium was dissolved in 500 ml of anhydrous ethanol, and after adding 23.8 g of ethyl acetoacetate thereto and stirring the solution at room temperature for 1 hour, 30 g of 3-chloromethylpyridine hydrochloride was added to the solution followed by heating mildly for 20 hours. After filtering away the salt formed, the solvent was distilled off under reduced pressure, and the residue formed was purified by column chromatography using chloroform — ethyl acetate as the developing solvent to provide 12.0 g of the oily product.

NMR (in $CDCl_3$)
δ(ppm); 1.18 (t, 3H)
2.20 (s, 3H)
3.13 (d, 2H)
3.78 (t, 1H)
4.13 (q, 2H)
7.04~7.60 (m, 2H)
840 (m, 2H)
Mass (m/z) 221 ($M^+$)

Reference Examples 2—8

By following the same procedure as in Reference Example 1, the following compounds were obtained:

Reference Example 2

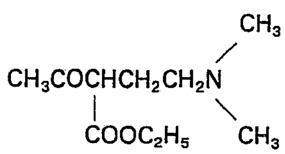

(NMR($CDCl_3$)
δ(ppm); 128 (t, 3H)
2.08 (q, 2H)
2.1~2.4 (11H)
3.58 (t,1H)
4.20 (q, 2H)
Mass (m/z) 201($M^+$), 156

Ethyl α-(2-N,N-dimethylaminoethyl)acetoacetate

Reference Example 3

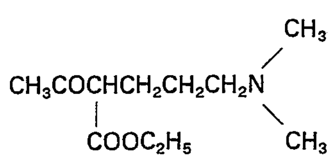

(NMR($CDCl_3$)
δ(ppm); 1.28 (t, 3H)
1.4~1.6 (m, 2H)
1.7~2.0 (m, 2H)
2.1~2.5 (11H)
3.44 (t, 1H)
4.19 (q, 2H)
Mass (m/z) 215($M^+$), 170

Ethyl α-(3-N,N-dimethylaminoethyl)acetoacetate

Reference Example 4

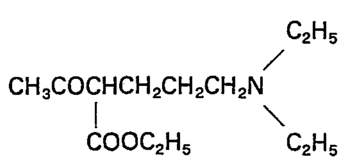

b.p. 110~114°C/(1.8 mmHg) 240Pa
Mass (m/z) 243 ($M^+$)
198 ($M^+$—45)

Ethyl α-3-diethylaminopropyl)acetoacetate

Reference Example 5

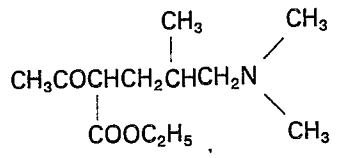

b.p. 75~78°C/(0.4 mmHg) 53 Pa
Mass (m/z) 229 ($M^+$)

Ethyl α-[3-(N,N-dimethylamino)-2-methylpropyl]-acetoacetate

Reference Example 6

(1)

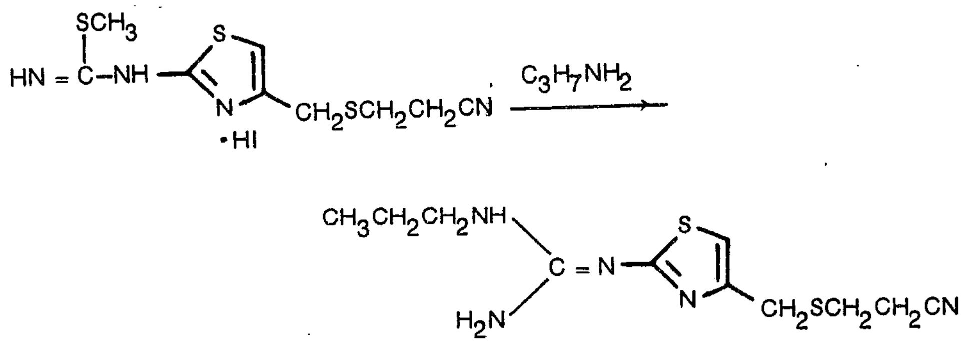


8.75 g of 3-[([2-(8-methylisothioureido)thiazole-4-yl]methyl]thio]propionitrile hydroiodide and 12.9 g of n-propylamine were dissolved in 200 ml of ethanol, and the solution was refluxed under heating for 48 hours. The solvent was distilled away, and the residue formed was purified by column chromatography using a mixture of chloroform and methanol as the developing solvent to provide 6.0 g of 3-(2-n-propylguanidinothiazol-4-ylmethylthio)propionitrile

(NMR($CDCl_3$)
δ(ppm); 1.03 (t, 3H)
1.68 (m, 2H)
2.4—2.9 (m, 4H)
3.20 (t, 2H)
3.72 (s, 2H)
6.44 (s, 1H)
Mass (m/z) 283 ($M^+$)

(2)

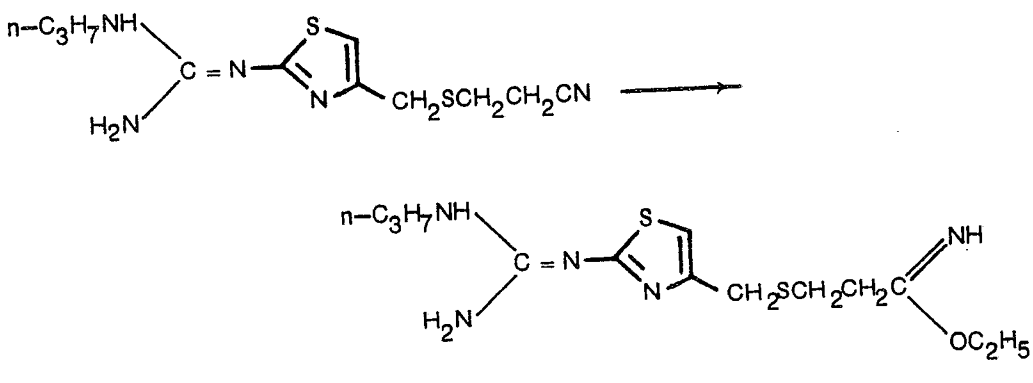

6.0 g of the nitrile compound obtained as in (1) above was dissolved in 100 ml of dry chloroform and after adding 20 ml of dry ethanol thereto and cooling the solution below 10°C and passing therethrough dry hydrogen chloride gas for 3 hours, the solution was allowed to stand at 0—4°C for 48 hours. Then, the solvents were distilled off and the residue was poured into a cooled aqueous solution of potassium carbonate for alkalifying, and the mixture was extracted with chloroform. The chloroform layer was washed with water and dried over anhydrous magnesium sulfate, and the solvent was distilled off under reduced pressure to provide 6.0 g of the oily product.

(3) 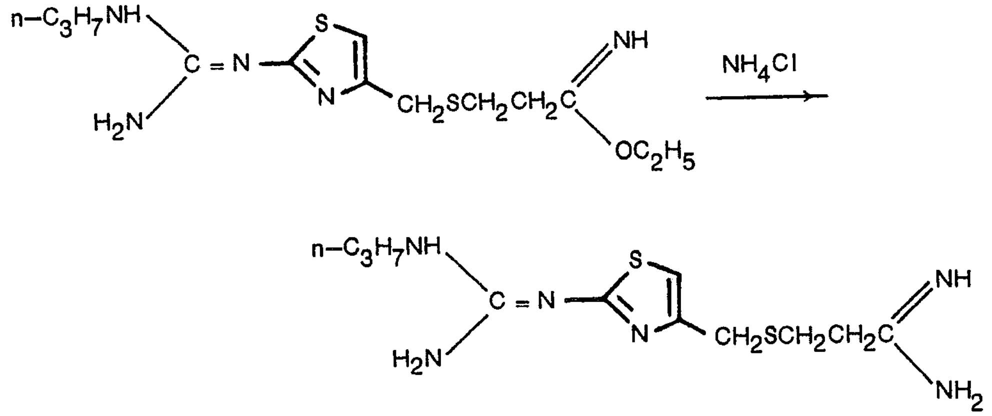


A mixture of 6.0 g of the imidate compound obtained as at (2) above and 1.0 g of ammonium chloride in 70 ml of methanol was stirred at room temperature for 1 hour, and the solvent was distilled off under reduced pressure. The residue was treated with hydrochloric acid in ethanol, and was recrystallized with acetonitrile — ethanol to provide 5.8 g of the amidine hydrochloride having a melting point of 183—184.5°C.

Reference Example 7

4-(m-piperidinomethylphenoxy)butaneamidine hydrochloride

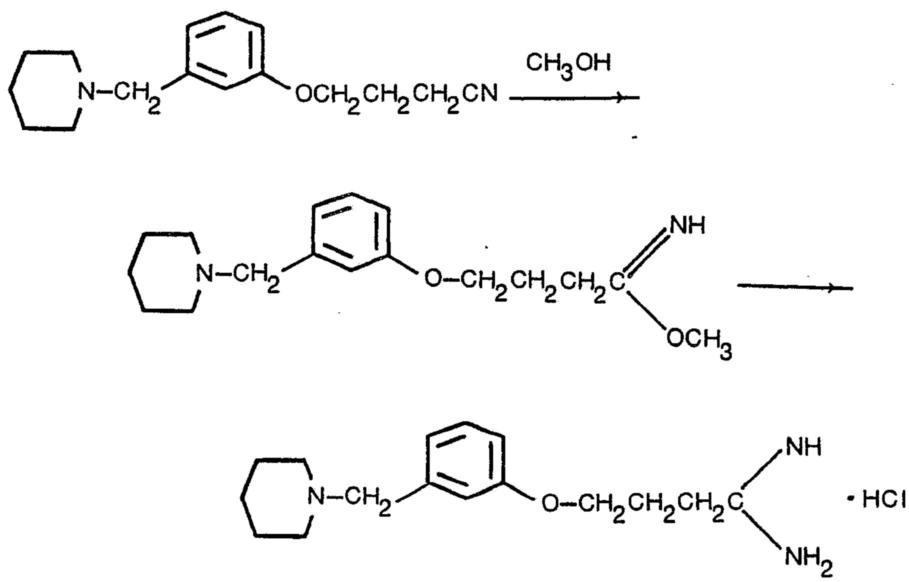


To 200 ml of dry chloroform were added 20.0 g (77.4 ml) of 4-(m-piperidinomethylphenoxy)-butanenitrile and 15.7 ml (387 mmol) of dry methanol, and the solution obtained was cooled by ice-water bath. Through the solution was passed hydrogen chloride gas at 10°C. After confirming the disappearance of the starting material in 8 hours, the reaction was stopped, and the solvent was distilled off under reduced pressure. The residue was dissolved in chloroform, and the solution was added to saturated aqueous potassium carbonate solution and stirred. The organic solvent layer was separated, and the aqueous layer was extracted with chloroform. The organic layer and the chloroform solution were combined, and the combined solution was dried over anhydrous magnesium sulfate. The solvent was distilled away to provide oily crude methyl 4-(m-piperidinomethylphenoxy)butaneimidate (21.1 g). The product was dissolved in 400 ml of dry methanol, and 3.5 g (65.4 mmol) of ammonium chloride was added to the obtained solution at room temperature. The solution became homogeneous in 10 minutes, and completion of the reaction was checked by thin layer chromatography. The imidate disappeared after stirring the solution for 3 hours (checked by chromatography). A small amount of precipitate was filtered away and the solution was concentrated to precipitate crystals. The precipitated crystals were collected by filtration to provide 12.3 g of colourless needle crystals.

The filtrate was concentrated to dryness. By recrystallization from ethyl acetate-ethanol, 5.6 g of product was obtained (18.0 mmol, 23%).

m.p. 151.0—152.0°C (ethanol-ethyl acetate)
Mass. (m/e) 275 ($M^+$—HCl)

Elemental analysis for $C_{16}H_{25}N_3O$ HCl

| | C | H | N | Cl |
|---|---|---|---|---|
| Calculated | 61.62 | 8.40 | 13.47 | 11.37 |
| Found | 61.47 | 8.51 | 13.52 | 11.41 |

Reference Example 8

3-[5-(dimethylaminomethyl)furfurylthiomethyl]propionamidine dihydrochloride

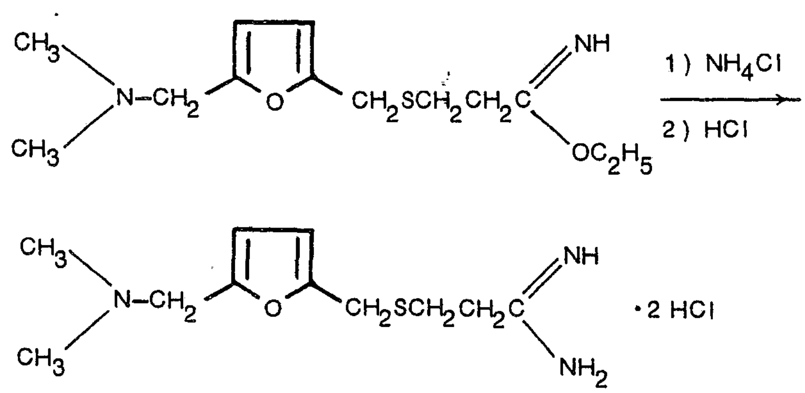


34 g of ethyl 3-[5-(dimethylaminomethyl)furfurylthiomethyl]pripionimidate was dissolved in 250 ml of dry methanol, and after adding 6 g of ammonium chloride and stirring the mixture at room temperature for 1 hour, an ethanol solution containing hydrochloric acid was added to the mixture to produce a dihydrochloride, and the solvent was distilled off. The residue was recrystallized from a mixture of isopropanol and ethyl acetate to provide 3-[5-(dimethylaminomethyl)furfurylthiomethyl]propionamidine di-hydrochloride (31 g) having a melting point of 134—136°C.

Standard Example A

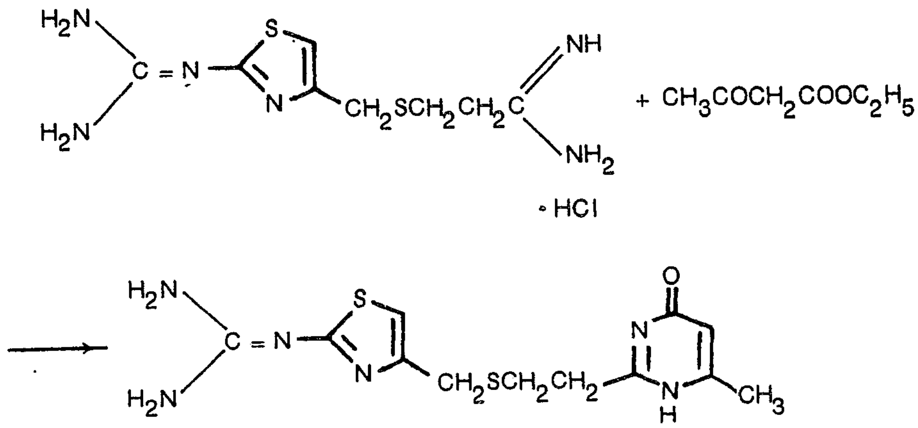

0.916 g of sodium methoxide (MeONa) was dissolved in 10 ml of methanol, and to the solution formed was added a solution of 5 g of 3-[[[2-[(diaminomethylene)amino]-4-thiazolyl]methyl]thio]propionamidine hydrochloride in 50 ml of methanol followed by stirring, and after adding 2.2 g of ethyl acetoacetate and stirring the mixture at room temperature for 20 hours, the salt formed was filtered away and the solvents were distilled off under reduced pressure. The residue was purified by column chromatography using chloroform — methanol as the developing solvent, and was recrystallized from ethanol to provide 2-[2-[[[2-[(diaminomethylene)amino]-4-thiazolyl]methyl]thio]ethyl]6-methyl-4-(1H)-pyrimidone (1.3 g) having a melting point of 176—177°C.

Anal. ($C_{12}H_{18}N_8OS_2$)

| | C | H | N |
|---|---|---|---|
| Calc. (%) | 44.43 | 4.97 | 25.90 |
| Found (%) | 44.39 | 4.97 | 25.86 |

By following the same procedure as in Standard Example A, the following compounds were obtained. (It is understood that the compounds exemplified in the following examples can be converted to the corresponding acid addition salt or free base as the case may be utilizing conventional methods.)

## Example 1

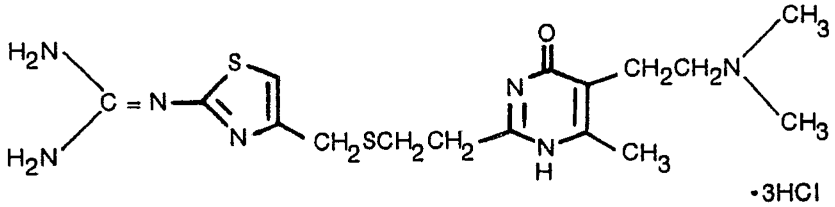

•3HCl

2-[2-[[[2-[(diaminomethylene)amino]-4-thiazolyl]methyl]thio]ethyl]-5-(2-dimethylaminoethyl)-6-methyl-4(1H)-pyrimidone trihydrochloride

m.p. 164~167°C
Anal. ($C_{10}H_{28}N_7OS_2Cl_3$)

| | C | H | N |
|---|---|---|---|
| Calc. (%) | 38.06 | 5.59 | 19.42 |
| Found(%) | 38.16 | 5.59 | 19.17 |

## Example 2

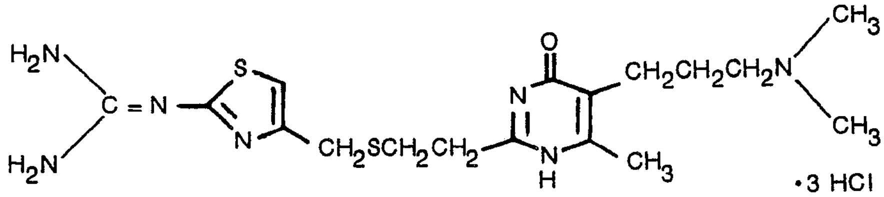

•3 HCl

2-[2-[[[2-[(diaminomethylene)amino]-4-thiazolyl]methyl]thio]ethyl]-5-(3-dimethylaminopropyl)-6-methyl-4(1H)-pyrimidone trihydrochloride
m.p. 175~177°C
Anal. ($C_{17}H_{30}N_7OS_2Cl_3 \cdot \frac{1}{2}H_2O$)

| | C | H | N |
|---|---|---|---|
| Calc. (%) | 38.67 | 5.92 | 18.57 |
| Found (%) | 38.38 | 5.90 | 18.46 |

Example 3

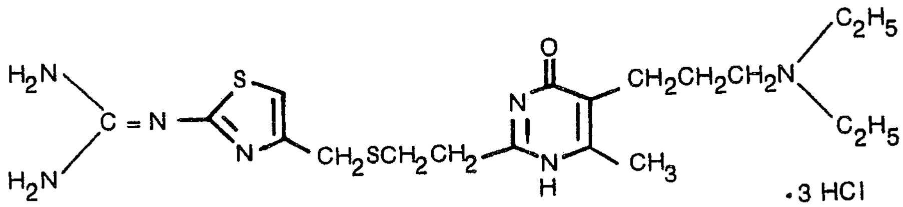

•3 HCl

2-[2-[[[2-[(diaminomethylene)amino]-4-thiazolyl]methyl]thio]ethyl]-5-(3-diethylaminopropyl)-6-methyl-4(1H)-pyrimidone trihydrochloride

m.p. 179~181°C (ethanol)
Anal. ($C_{19}H_{34}N_7OS_2Cl_3$)

| | C | H | N | S | Cl |
|---|---|---|---|---|---|
| Calc. (%) | 41.72 | 6.26 | 17.92 | 11.72 | 19.44 |
| Found (%) | 41.68 | 6.47 | 17.82 | 11.63 | 19.41 |

Example 4

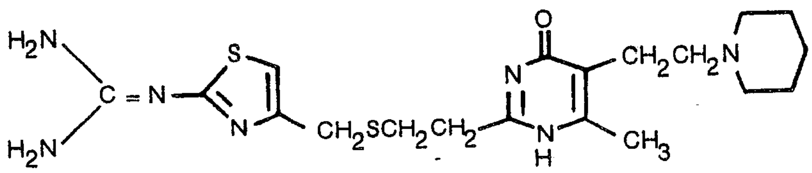

2-[2-[[[2-[(diaminomethylene)amino]-4-thiazolyl]methyl]thio]ethyl]-6-methyl-5-(2-piperidinoethyl)-4(1H)-pyrimidone tri-hydrochloride

m.p. 156~159°C (ethanol).3HCl
Anal. ($C_{19}H_{32}N_7OS_2Cl_3.\frac{1}{2}H_2O$)

| | C | H | N |
|---|---|---|---|
| Calc. (%) | 41.19 | 6.00 | 17.70 |
| Found (%) | 41.04 | 6.15 | 17.80 |

Example 5

2-[2-[[[2-[(diaminomethylene)amino]-4-thiazolyl]methyl]thio]ethyl]-6-methyl-5-(2-methyl-3-dimethylaminopropyl)-4(1H)-pyrimidone tri-hydrochloride

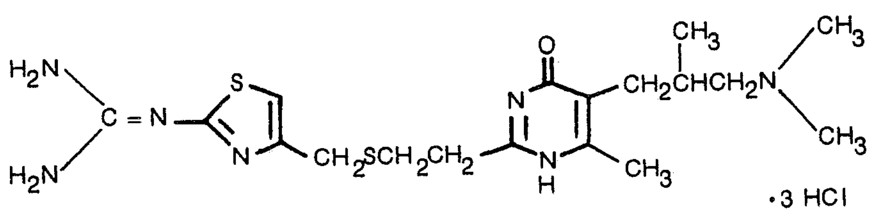

•3 HCl

m.p. 194~198°C
Anal. ($C_{15}H_{32}N_7OS_2Cl_3$)

| | C | H | N | S | Cl |
|---|---|---|---|---|---|
| Calc. (%) | 40.56 | 6.05 | 18.40 | 12.03 | 19.96 |
| Found (%) | 40.57 | 6.27 | 18.56 | 12.02 | 19.93 |

Example 6

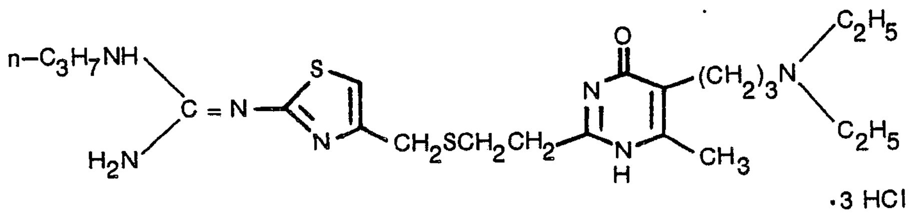

5-(3-diethylaminopropyl)-6-methyl-2-[2-[[[2-[[n-propyl diaminomethylene]amino]-4-thiazolyl]methyl]thio]-ethyl]4(1H)-pyrimidone trihydrochloride

m.p. 184~187°C
Anal. ($C_{22}H_{40}N_7OS_2Cl_3$)

| | C | H | N | S | Cl |
|---|---|---|---|---|---|
| Calc. (%) | 44.86 | 6.84 | 16.64 | 10.88 | 18.06 |
| Found (%) | 44.85 | 7.13 | 16.62 | 10.97 | 18.07 |

Mass. (m/z) 479 ($M^+$)

Standard Example B

6-Methyl-2-methyl-3-(m-piperidinomethylphenoxy)propyl-4(1H)-pyrimidone

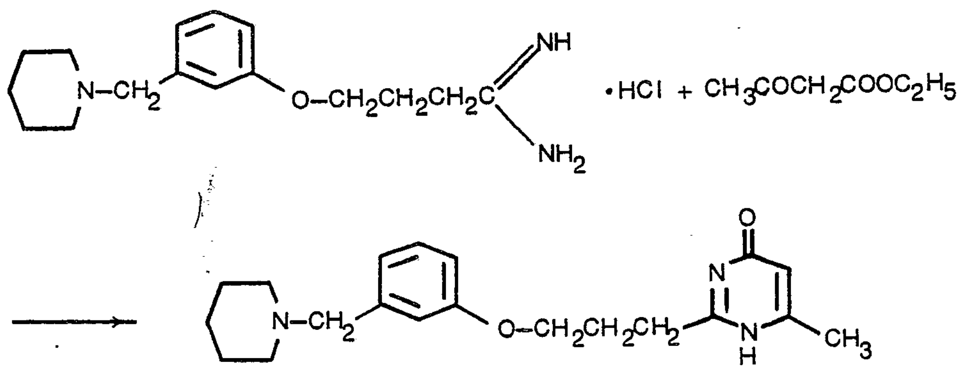


To 30 ml of dry ethanol was added metallic sodium under cooling. To the resulting solution was added gradually a solution of 3.5 g of 4-(m-piperidinomethylphenoxy)butaneamidine hydrochloride in 10 ml of dry methanol under cooling, followed by stirring for 10 minutes. Then, to the solution was added gradually a solution of 1.28 ml of ethyl acetoacetate in 10 ml of dry ethanol under cooling. After stirring for 24 hours at room temperature, the resulting salt was filtered and the solvent was distilled off under reduced pressure. The residue obtained was purified by column chromatography using chloroform-methanol as a developing solvent, and was recrystallized from iso-propanol to provide 2.04 g of product.

m.p. 120~121°C (iso-propanol)
Mass. 341 ($M^+$)
Anal. ($C_{20}H_{27}N_3O_2$)

| | C (%) | H (%) | N (%) |
|---|---|---|---|
| Calc. | 70.35 | 7.97 | 12.31 |
| Found | 70.62 | 7.92 | 12.45 |

By following the same procedure as in Standard Example B, the following compound was obtained:

Example 7

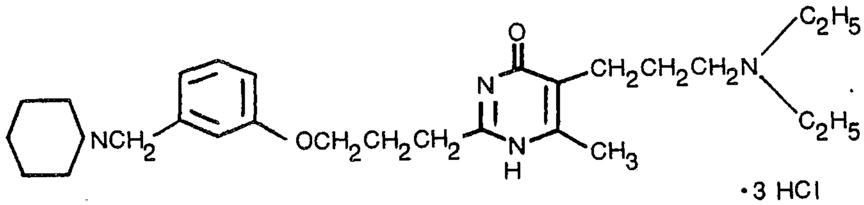

mp. 128—130°C
Mass (m/z) 454 ($M^+$), 425 ($M^+$—29)

## Claims

1. A compound of the formula I or a pharmaceutically acceptable salt thereof

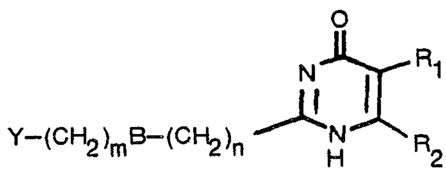
I

wherein $R_1$ represents the group

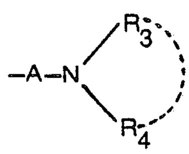

wherein A represents a $C_1$ to $C_5$ alkylene group and $R_3$ and $R_4$ are the same or different $C_1$ to $C_5$ alkyl groups or combine to form with the adjacent nitrogen atom a piperidine or pyrrolidine ring; $R_2$ represents a hydrogen atom, a $C_1$ to $C_5$ alkyl group or a trifluoromethyl group; Y represents a heterocyclic group of the formula:

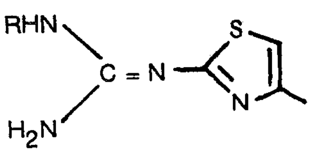
,
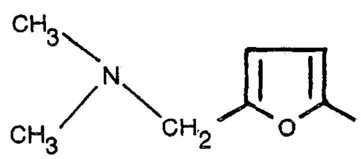

or
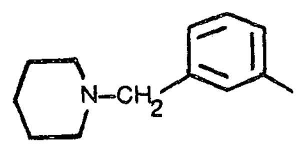

wherein R represents a hydrogen atom or a $C_1$ to $C_5$ alkyl group; B represents an oxygen atom or a sulfur atom; m represents zero or an integer of 1 to 3; and n represents an integer of 1 to 3.

2. A compound according to claim 1 wherein B is oxygen and m is zero.

3. A compound according to claim 1 wherein B is sulfur and m and n are the same or different integers of 1 to 3.

4. A compound according to claim 1 wherein Y is

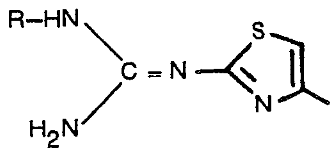

B is sulfur, and m and n are the same or different integers of 1 to 3.

5. A compound according to claim 4 wherein R is a $C_1$ to $C_3$ alkyl group.

6. A compound according to claim 1 wherein Y is

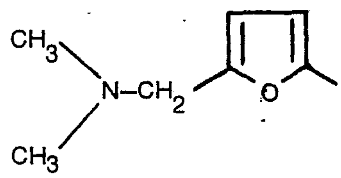

and m and n are the same or different integers of 1 to 3.

7. A compound according to claim 1 wherein Y is

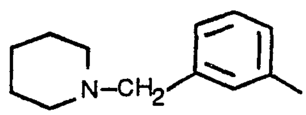

B is oxygen, and m is zero.

8. At least one compound according to claim 1 selected from

2-[2-[[[2-[(diaminomethylene)amino]-4-thiazolyl]methyl]thio]ethyl]-5-(3-dimethylaminopropyl)-6-methyl-4(1H)-pyrimidone;

2-[2-[[[2-[(diaminomethylene)amino]-4-thiazolyl]methyl]thio]ethyl]-6-methyl-5-(2-methyl-3-dimethylaminopropyl)-4(1H)-pyrimidone;

5-(3-diethylaminopropyl)-6-methyl-2-[2-[[[2-[[n-propyldiaminomethylene]amino]-4-thiazolyl]methyl]thio]ethyl]-4(1H)-pyrimidone;

and pharmaceutically acceptable salts thereof.

9. A compound according to claim 1 which is 2-[2-[[[2-[(diaminomethylene)amino]-4-thiazolyl]methyl]-thio]ethyl]-5-(3-diethylaminopropyl)-6-methyl-4(1H)-pyrimidone or a pharmaceutically acceptable salt thereof.

10. A pharmaceutical composition containing a compound according to any preceding claim and excipient.

11. A process for preparing a compound of formula I

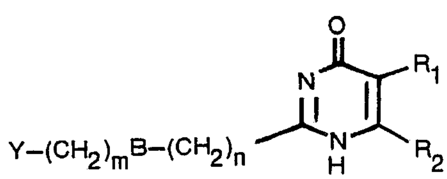

I

which comprises reacting a compound of formula:

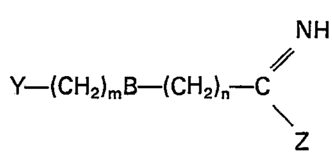

with a compound of the formula:

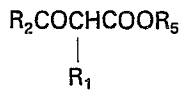 or 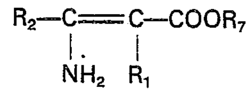

wherein $R_1$ represents the group

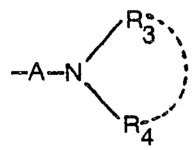

wherein A represents a $C_1$ to $C_5$ alkylene group and $R_3$ and $R_4$ are the same or different $C_1$ to $C_5$ alkyl groups or combine to form with the adjacent nitrogen atom a piperidine or pyrrolidine ring; $R_2$ represents a hydrogen atom, a $C_1$ to $C_5$ alkyl group or a trifluoromethyl group; Y represents a heterocyclic group of the formula:

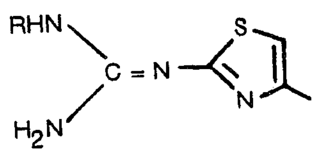 , 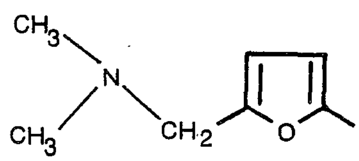

or 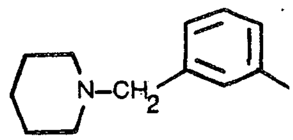

wherein R represents a hydrogen atom or a $C_1$ to $C_5$ alkyl group; B represents an oxygen atom or a sulfur atom; m represents zero or an integer of 1 to 3; and n represents an integer of 1 to 3; $R_5$ is a $C_1$ to $C_5$ alkyl group, $R_7$ is a $C_1$ to $C_5$ alkyl group; and Z is $NH_2$ or $OR_6$ wherein $R_6$ is a $C_1$ to $C_5$ alkyl group.

12. A process according to claim 11 which comprises reacting a compound of the formula:

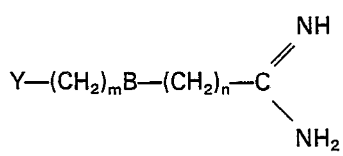

wherein Y, B, m and n are as defined in claim 11 with a compound of the formula

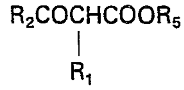

wherein $R_1$, $R_2$ and $R_5$ are as defined in claim 11.

13. A process according to claim 11 which comprises reacting a compound of the formula:

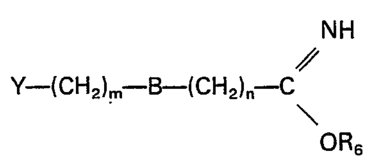

wherein Y, B, $R_6$, m and n are as defined in claim 11 with a compound of the formula:

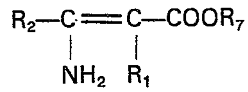

wherein $R_1$, $R_2$, and $R_7$ are as defined in claim 11.

14. A process according to claim 11, 12 or 13 including the step of converting the free base product to acid addition salt or vice-versa.

**Patentansprüche**

1. Eine Verbindung der Formel I oder ein pharmazeutisch annehmbares Salz derselben

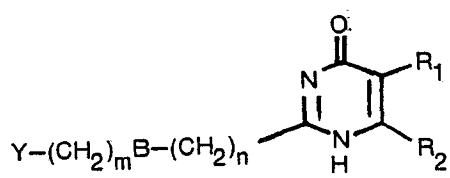

I

worin $R_1$ die

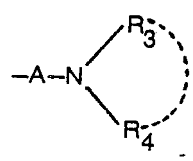

-Gruppe bedeutet, worin A eine $C_1$ bis $C_5$ Alkylengruppe bedeutet und $R_3$ und $R_4$ dieselbe oder verschiedene $C_1$ bis $C_5$ Alkylgruppen bedeuten oder zusammen mit dem banachbarten Stickstoffatom zu einem Piperidin- oder Pyrrolidinring kombiniert sind $R_2$ bedeutet ein Wasserstoffatom, eine $C_1$ bis $C_5$ Alkylgruppe oder eine Trifluoromethylgruppe; Y bedeutet eine heterocyklische Gruppe der Formel

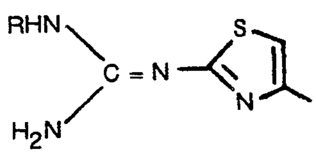

,

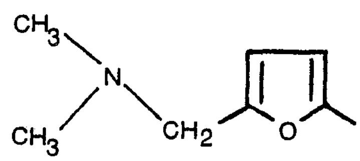

oder

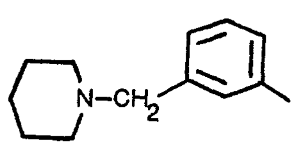

worin R ein Wasserstoffatom oder eine $C_1$ bis $C_5$ Alkylgruppe bedeutet; B bedeutet ein Sauerstoff- oder ein Schwefelatom; m bedeutet Null oder eine ganze Zahl von 1 bis 3; und n bedeutet eine ganze Zahl von 1 bis 3.

2. Eine Verbindung nach Anspruch 1, worin B Sauerstoff und m Null ist.

3. Eine Verbindung nach Anspruch 1, worin B Schwefel und m und n dieselben oder verschiedene ganze Zahlen von 1 bis 3 sind.

4. Eine Verbindung nach Anspruch 1, worin Y

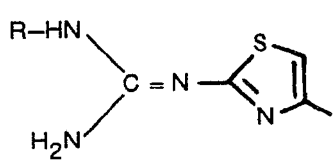

,

ist, B ist Schwefel und m und n sind dieselbe oder verschiedene der ganzen Zahlen von 1 bis 3.

5. Eine Verbindung nach Anspruch 4, worin R eine $C_1$ bis $C_3$ Alkylgruppe ist.

6. Eine Verbindung nach Anspruch 1, worin Y

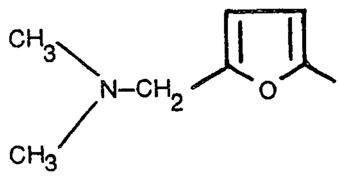

ist, und m und n dieselben oder Verschiedene ganze Zahlen von 1 bis 3 sind.

7. Eine Verbindung nach Anspruch 1, worin Y

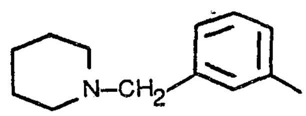

ist, B ist Sauerstoff und m ist Null.

8. Wenigstens eine Verbindung nach Anspruch 1, ausgewählt aus

2-[2-[[[2-[(Diaminomethylen)amino]-4-thiazolyl]methyl]thio]äthyl]-5-(3-dimethylaminopropyl)-6-methyl-4(1H)-pyrimidon;

2-[2-[[[2-[(Diaminomethylen)amino]-4-thiazolyl]methyl]thio]äthyl]-6-methyl-5-(2-methyl-3-dimethylaminopropyl)-4-(1H)-pyrimidon;

5-(3-Diäthylaminopropyl)-6-methyl-2-[2-[[[2-[(n-propyldiaminomethylen)-amino]-4-thiazolyl]methyl-thio]äthyl]-4(1H)-pyrimidon; und deren pharmazeutisch annehmbaren salze.

9. Eine Verbindung nach Anspruch 1, welche 2-[2-[[[2-[(Diaminomethylen)amino]-4-thiazolyl]methyl]-thio]äthyl]-5-(3-diäthylaminopropyl)-6-methyl-4(1H)-pyrimidon oder eine pharmazeutisch annehmbares Salz derselben ist.

10. Eine pharmazeutische Zusammensetzung, enthaltend eine Verbindung nach einem der vorhergehenden Ansprüche und ein Hilfsmittel.

11. Ein Verfahren zur Herstellung einer Verbindung der Formel I

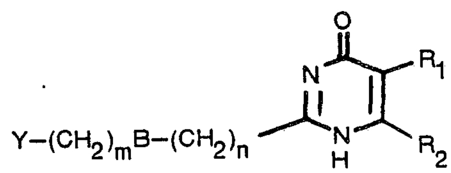

I

dadurch gekennzeichnet, daß man eine Verbindung der Formel

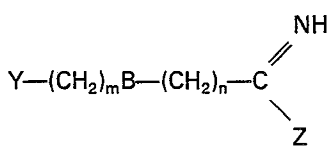

mit einer Verbindung der Formel

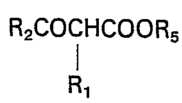 oder 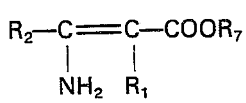

worin $R_1$ die

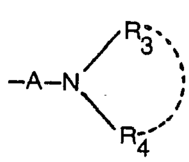

-Gruppe bedeutet, worin A eine $C_1$ bis $C_5$ Alkylengruppe und $R_3$ und $R_4$ die gleiche oder verschiedene $C_1$ bis $C_5$ Alkylgruppen sind oder zusammen mit dem benachbarten Stickstoffatom zu einem Piperidin- oder Pyrrolidinring kombiniert sind; $R_2$ bedeutet ein Wasserstoffatom, eine $C_1$ bis $C_5$ Alkylgruppe oder eine Trifluoromethylgruppe; Y bedeutet eine heterozyklische Gruppe der Formel:

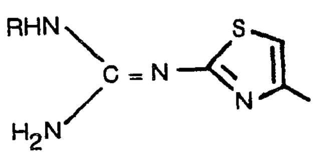 , 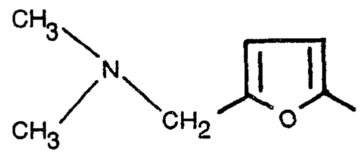

oder 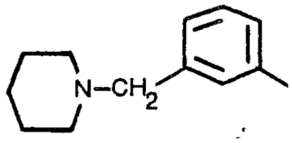

worin R ein Wasserstoffatom oder eine $C_1$ bis $C_5$ Alkylgruppe; B ein Sauerstoffatom oder ein Schwefelatom bedeutet, m bedeutet Null oder eine ganze Zahl von 1 bis 3; und n bedeutet eine ganze Zahl von 1 bis 3; $R_5$ ist eine $C_1$ bis $C_5$ Alkylgruppe; $R_7$ ist eine $C_1$ bis $C_5$ Alkylgruppe; und Z ist $NH_2$ oder $OR_6$, worin $R_6$ eine $C_1$ bis $C_5$ Alkylgruppe ist.

12. Verfahren nach Anspruch 11, dadurch gekennzeichnet, daß man eine Verbindung der Formel

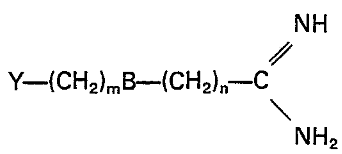

worin Y, B, m und n wie in Anspruch 11 definiert sind, mit einer Verbindung der Formel

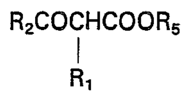

worin $R_1$, $R_2$ und $R_5$ wie in Anspruch 11 definiert sind, umsetzt.

13. Verfahren nach Anspruch 11, dadurch gekennzeichnet, daß man eine Verbindung der Formel

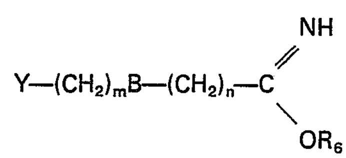

worin Y, B, $R_6$, m und n wie in Anspruch 11 definiert sind, mit einer Verbindung der Formel

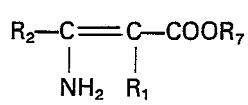

worin $R_1$, $R_2$ und $R_7$ wie in Anspruch 11 definiert sind, umsetzt.

14. Verfahren nach Anspruch 11, 12 oder 13, dadurch gekennzeichnet, daß man die freie Base in das Säureadditionssalz umwandelt oder umgekehrt.

**Revendications**

1. Composé de la formule I ou sel pharmaceutiquement acceptable de celui-ci

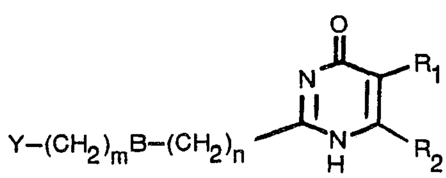 I

dans laquelle $R_1$ représente le groupe

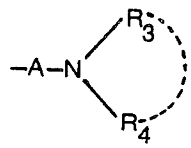

dans lequel A représente un groupe alkylène en $C_1$ à $C_5$ et $R_3$ et $R_4$ sont des groupes alkyles en $C_1$ à $C_5$ identiques ou différents ou se combinent pour former avec l'atome d'azote adjacent un noyau pipéridine ou pyrrolidine; $R_2$ représente un atome d'hydrogène, un groupe alkyle en $C_1$ à $C_5$ ou un groupe trifluorométhyle; Y représente un groupe hétérocyclique de la formule:

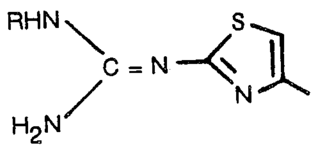

,

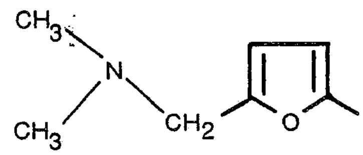

ou

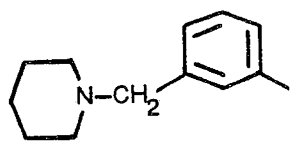

dans laquelle R représente un atome d'hydrogène ou un groupe alkyle en $C_1$ à $C_5$; B représente un atome d'oxygène ou un atome de soufre; *m* représente zéro ou un nombre entier de 1 à 3; et *n* représente un nombre entier de 1 à 3.

2. Composé selon la revendication 1, dans lequel B est l'oxygène et *m* est zéro.

3. Composé selon la revendication 1, dans lequel B est le soufre et *m* et *n* sont des nombres entiers identiques ou différents de 1 à 3.

4. Composé selon la revendication 1, dans lequel Y est

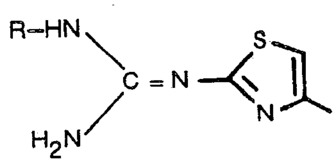

,

B est le soufre et *m* et *n* sont des nombres entiers identiques ou différents de 1 à 3.

5. Composé selon la revendication 4, dans lequel R est un groupe alkyle en $C_1$ à $C_3$.

6. Composé selon la revendication 1, dans lequel Y est

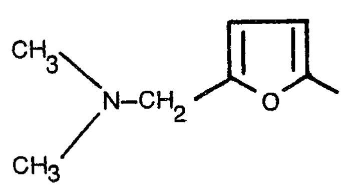

et *m* et *n* sont des nombres entiers identiques ou différents de 1 à 3.

7. Composé selon la revendication 1, dans lequel Y est

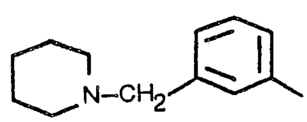

B est l'oxygène et *m* est zéro.

8. Au moins un composé selon la revendication 1, choisi entre

la 2-[2-[[[2-[(diaminométhylène)amino]-4-thiazolyl]méthyl]thio]éthyl]-5-(3-diméthylaminopropyl)-6-méthyl-4(1H)-pyrimidone;

la 2-[2-[[[2-[(diaminométhylène)amino]-4-thiazolyl]méthyl]thio]éthyl]-6-méthyl-5-(2-méthyl-3-diméthylaminopropyl)-4(1H)-pyrimidone;

la 5-(3-diéthylaminopropyl)-6-méthyl-2-[2-[[[2-[[n-propyldiaminométhylène]-amino]-4-thiazolyl]méthyl]thio]éthyl]-4(1H)pyrimidone; et leurs seuls pharmaceutiquement acceptables.

9. Composé selon la revendication 1, qui est la 2-[2-[[[2-[(diaminométhylène)amino]-4-thiazolyl]-méthyl]-thio]éthyl]-5-(3-diéthylaminopropyl)-6-méthyl-4(1H)-pyrimidone ou un sel pharmaceutiquement acceptable de celle-ci.

10. Composition pharmaceutique contenant un composé selon l'une quelconque des revendications précédentes et un excipient.

11. Procédé pour la préparation d'un composé de formule I

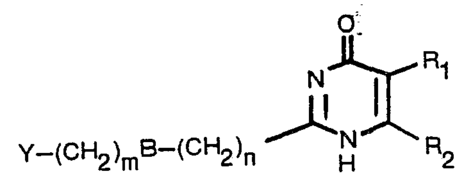

I

qui consiste à faire réagir un composé de formule:

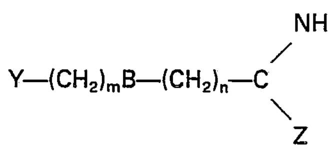

sur un composé de la formule:

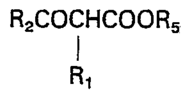

ou

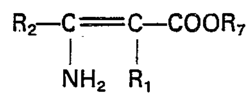

dans lesquelles $R_1$ représente le groupe

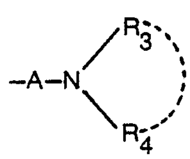

dans lequel A représente un groupe alkylène en $C_1$ à $C_5$ et $R_3$ et $R_4$ sont des groupes alkyles en $C_1$ à $C_5$ identiques ou différents ou se combinent pour former avec l'atome d'azote adjacent un noyau pipéridine ou pyrrolidine; $R_2$ représente un atome d'hydrogène, un groupe alkyle en $C_1$ à $C_5$ ou un groupe trifluorométhyle; Y représente un groupe hétérocyclique de la formule:

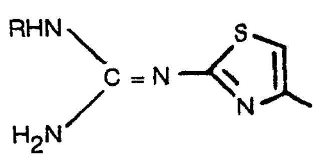

,

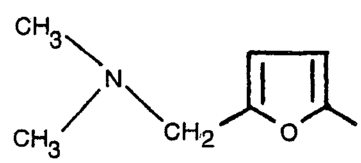

ou

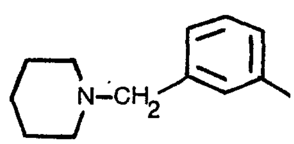

dans laquelle R représente un atome d'hydrogène ou un groupe alkyle en $C_1$ à $C_5$; B représente un atome d'oxygène ou un atome de soufre; *m* representé zéro ou un nombre entier de 1 à 3 et *n* représente un nombre entier de 1 à 3; $R_5$ est un groupe alkyle en $C_1$ à $C_5$, $R_7$ est un groupe alkyle en $C_1$ à $C_5$; et Z est $NH_2$ ou $OR_6$ dans lequel $R_6$ est un groupe alkyle en $C_1$ à $C_5$.

12. Procédé selon la revendication 11, qui consiste à faire réagir un composé de la formule:

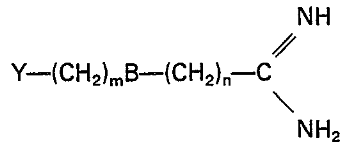

dans laquelle Y, B, *m* et *n* sont tels que définis à la revendication 11, sur un composé de la formule

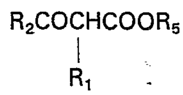

dans laquelle $R_1$, $R_2$ et $R_5$ sont tels que définis à la revendication 11.

13. Procédé selon la revendication 11, qui consiste à faire réagir un composé de la formule:

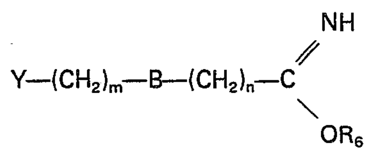

dans laquelle Y, B, $R_6$, *m* et *n* sont tels que définis à la revendication 11, sur un composé de la formule:

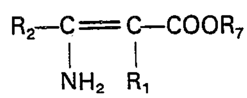

dans laquelle $R_1$, $R_2$ et $R_7$ sont tels que définis à la revendication 11.

14. Procédé selon l'une des revendications 11, 12 ou 13, comprenant l'étape consistant à convertir la base libre obtenue comme produit en sel d'addition d'acide ou vice versa.